# EUROPEAN PATENT APPLICATION

(11) **EP 2 508 620 A1**
(43) Date of publication of application: **10.10.2012**
(21) Application number: 11161675.1
(22) Date of filing: 08.04.2011
(51) Int. Cl.: C12Q 1/68, C12Q 1/70, B01L 7/00

(54) **Viral load assessment in multiple HPV infections as predictor for the presence of cervical lesions**

(71) Applicant: Deutsches Krebsforschungszentrum, 69120 Heidelberg (DE)
(72) Inventor: The designation of the inventor has not yet been filed
(74) Representative: Dick, Alexander

(57) **Abstract**

The present invention is concerned with a method for differentiating in a subject infected with at least two high-risk (HR-) human papillomavirus (HPV) genotypes between a severe form of HPV infection and a mild form of HPV infection, comprising a) quantifying the genome numbers of each of said at least two HR-HPV-genotypes in a sample from said patient, b) comparing the genome number of the HR-HPV genotype with the highest genome number to at least one reference amount, and c) differentiating between (i) a severe form of HPV infection and (ii) a mild form of HPV infection. Furthermore, the current invention relates to a method for diagnosing a severe form of HPV infection in a subject comprising the method of the invention, as well as to oligonucleotides for use in said method for diagnosis and a device for use in said method.

## Description

The present invention is concerned with a method for differentiating in a subject infected with at least two high-risk (HR-) human papillomavirus (HPV) genotypes between a severe form of HPV infection and a mild form of HPV infection, comprising a) quantifying the genome numbers of each of said at least two HR-HPV-genotypes in a sample from said patient, b) comparing the genome number of the HR-HPV genotype with the highest genome number to at least one reference amount, and c) differentiating between (i) a severe form of HPV infection and (ii) a mild form of HPV infection. Furthermore, the current invention relates to a method for diagnosing a severe form of HPV infection in a subject comprising the method of the invention, as well as to oligonucleotides for use in said method for diagnosis and a device for use in said method.

Cervical cancer (cancer of the uterine cervix) is the second most common cancer among women worldwide with about 470,000 newly diagnosed cases and almost 250,000 deaths every year (Parkin, DM et al. 2001. Eur. J. Cancer 37 (Suppl. 8):4-66). The predominant cause for cervical cancer is infection of the cervix with human papillomavirus (HPV), particularly with high-risk HPV genotypes. Human papillomaviruses form a large group of viruses and are small, non-enveloped DNA viruses that infect almost exclusively skin and mucosal cells. To date, the genomes of almost 100 various genotypes of human papillomaviruses have been characterized (de Villiers, E. M., C. Fauquet, T. R. Broker, H. U. Bernard, and H. zur Hausen. 2004. Classification of papillomaviruses. Virology 324:17-27), and a much larger number of human papillomaviruses is thought to exist (Hazard K, Andersson K, Dillner J, Forslund O. Human papillomavirus subtypes are not uncommon. Virology. 2007 May 25;362(1):6-9).

Approximately 50 HPV genotypes are known to infect the mucosa. These mucosal genotypes are classified into three different groups based on their epidemiological association with cancer: "low-risk" human papillomaviruses (LR-HPV), "high-risk" human papillomaviruses (HR-HPV) and "putative high-risk" human papillomaviruses (pHR-HPV). Low-risk human papillomaviruses (including, e.g., HPV genotypes 6, 11, 40, 42, 43 and 44) are primarily found in genital warts and low-grade cervical lesions. Possibly high-risk human papillomaviruses, including e.g. HPV 26, 53 and 70, have not consistently been found in cervical cancer. High-risk human papillomaviruses are associated with the risk of developing cancer (Munoz et al. 2003. N. Engl. J. Med. 348:518-527) and include HPV 16, 18, 31, 33, 35, 39, 45, 51, 52, 56, 58, 59, 66 and 68, HPV 16 and 18 are considered the clinically most relevant ones, as these genotypes are found in almost 70 % of cervical cancer patients.

Infection with a high-risk HPV genotype (HR-HPV) does not necessarily lead to cervical cancer. However, several studies clearly have shown that women infected with high-risk HPV are at substantially higher risk of developing cancer than uninfected women or women being infected with low-risk HPV (Bosch, F. X., M. M. Manos, N. Munoz, M. Sherman, A. M. Jansen, J. Peto, M. H. Schiffman, V. Moreno, R. Kurman, and K. V. Shah. 1995. Prevalence of human papillomavirus in cervical cancer: a worldwide perspective. International biological study on cervical cancer (IBSCC) Study Group. J Natl Cancer Inst 87:796-802.; Bosch, F. X., A. Lorincz, N. Munoz, C. J. Meijer, and K. V. Shah. 2002. The causal relation between human papillomavirus and cervical cancer. J Clin Pathol 55:244-65). In addition, follow-up studies of women with and without cervical abnormalities have indicated that persistence of high-risk HPV infection is a significant risk factor and necessary for the development, maintenance and progression of cervical intraepithelial neoplasia (CIN) (Nobbenhuis, M. A., J. M. Walboomers, T. J. Helmerhorst, L. Rozendaal, A. J. Remmink, E. K. Risse, H. C. van der Linden, F. J. Voorhorst, P. Kenemans, and C. J. Meijer. 1999. Relation of human papillomavirus status to cervical lesions and consequences for cervical-cancer screening: a prospective study. Lancet 354:20-5).

Generally, the progression from HPV infection to cancer is very slow and may take years. However, a crucial step for a successful prevention of cervical cancer is the early detection of an infection with a high-risk HPV genotype. The introduction of the Papanicolaou test, frequently also referred to as Pap test or Pap smear, for cervical cancer screening led to a substantial decrease of mortality caused by cervical cancer in most industrialized countries. For the Papanicolaou test, samples are obtained from the cervix and screened by light microscopy for changes in cell morphology indicating malignant or premalignant cells. Then, samples are classified depending on the severity of the observed lesions. However, diagnosis by cervical cytology is a subjective method, and the quality depends on the standards of the laboratory that provides the service. In fact, sensitivity of cytology was shown to vary between 20 and 90 % in different studies (compared to colposcopy and histology as gold standard). This is in marked contrast to the diagnosis by detection of HPV DNA (sensitivity > 90 % in all studies, average 96 %) where commercial kits and standardized protocols were used. Moreover, specific HPV genotypes or even multiple infections with various HPV genotypes can not be identified. The identification of specific HPV genotypes, however, is important, as various HPV genotypes may pose different risks to the affected patients.

Therefore, new test systems were developed in order to allow the identification of specific HPV genotypes. These new test systems are almost exclusively based on the detection of viral, molecular and biochemical markers, such as HPV DNA and RNA. Recently, a test system has been developed that allows simultaneous detection of several HR-HPV genotypes in a single reaction (WO/2009/027403), which is of importance since approximately 50% of women infected with HPV bear infections with two or more HR-HPV genotypes (multiple HPV infections) (Schmitt, M., B. Dondog, T. Waterboer, M. Pawlita, M. Tommasino, and T. Gheit. 2009. Abundance of multiple high-risk HPV infections in cervical cells analyzed by an ultra-sensitive HPV genotyping assay. J Clin Microbiol; 48:143-149).

Despite the ability to detect multiple HPV infections in cervix samples, to date, there is no consistent method of assessing the risk of having severe cervical lesions or cervical cancer associated with such multiple HPV infections. For this reason, samples with multiple HPV infections are often excluded from studies (Hesselink AT, Berkhof J, Heideman DA, Bulkmans NW, van Tellingen JE, Meijer CJ, Snijders PJ. High-risk human papillomavirus DNA load in a population-based cervical screening cohort in relation to the detection of high-grade cervical intraepithelial neoplasia and cervical cancer. International journal of cancer 2009;124:381-6). If cases of multiple HPV infections are included, use of a composite viral load measurement is the state of the art, meaning that the amount of HR-HPV genomes is summed up for analysis, irrespective of the contribution of the individual genotypes to this sum. This can be achieved by design of the test method, like in the hybrid capture test, which does not discriminate among the HR-HPV genotypes in an a sample since it provides an overall signal as a readout which is the sum of all HR-HPV genotypes detectable in the test (Castle PE, Schiffman M, Wheeler CM. Hybrid capture 2 viral load and the 2-year cumulative risk of cervical intraepithelial neoplasia grade 3 or cancer. American journal of obstetrics and gynecology 2004; 191:1590-7). Alternatively, as it was proposed in the context of a qualitative assay, the signal strength categories are summed for the different HR-HPV genotypes (Gravitt et al. (2007): High load for most high risk human papillomavirus genotypes is associated with prevalent cervical cancer precursors but only HPV16 load predicts the development of incident disease. Int J Cancer: 121, 2787). In that study, it was found that composite viral load measurement gave a good correlation with prevalent CIN 2 or worse; however, in women infected with HPV16, concomitant infection with other genotypes would not increase risk.

Also, it would be desirable if all stages of CIN could be diagnosed. Even though CIN 1 lesions frequently regress spontaneously, it may nonetheless be of value for the patient to examine the lesion more closely and potentially even remove the affected area. It is recommended to at least shorten the interval between preventive examinations in case CIN 1 has been diagnosed.

The technical problem underlying the present invention, therefore, can be seen as the provision of means and methods for reliably diagnosing severe or mild HPV infections. The technical problem is solved by the embodiments characterized in the claims and herein below.

Accordingly, the current invention relates to a method for differentiating in a subject infected with at least two high-risk (HR-) human papillomavirus (HPV) -genotypes between a severe form of HPV infection and a mild form of HPV infection, comprising a) quantifying the genome numbers of each of said at least two HR-HPV-genotypes in a sample from said patient, b) comparing the genome number of the HR-HPV genotype with the highest genome number to at least one reference amount, and c) differentiating between (i) a severe form of HPV infection and (ii) a mild form of HPV infection.

The method of the present invention, preferably, is an in vitro method. Moreover, it may comprise steps in addition to those explicitly mentioned above. For example, further steps may relate to sample pre-treatments or evaluation of the results obtained by the method. The method of the present invention preferably is used for differentiating between a mild and a severe form of HR-HPV infection in subjects being infected with at least two genotypes of HR-HPV. However, the method of the present invention may also be used for monitoring, confirmation, and sub-classification of said subject. The method may be carried out manually or assisted by automation. Preferably, steps a) and / or b) and / or c) may in total or in part be assisted by automation, e.g., by a suitable robotic and sensory equipment for the quantification in step a), or a computer-implemented calculation or comparison step in steps b) and / or c).

The term "differentiating" as used herein means to distinguish between a severe form of HPV infection and a mild form of HPV infection. The term as used herein, preferably, includes differentially diagnosing or detecting a mild and a severe form of HR-HPV infection.

As will be understood by those skilled in the art, the aforementioned differentiation is usually not intended to be correct for 100% of the subjects to be analyzed. The term, however, requires that the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.001. Preferably, the probability envisaged by the present invention allows that the differentiation will be correct for at least 60%, at least 70%, at least 80%, at least 85%, at least 90%, at least 95% or at least 97% of the subjects of a given cohort.

It is thus, preferably, envisaged by the current invention that the differentiation results in the knowledge of how probable it is that a given subject is inflicted with a severe form of HPV infection. Hence, the risk of said subject to suffer from a severe form of HPV infection is estimated, where a genome number higher than the reference amount for at least one HR-HPV-type is indicative of a high risk of suffering from a severe form of HPV infection. Advantageously, it was found in the studies underlying this specification, that detection of genome numbers above the reference amount for more than one HR-HPV genotype is indicative of a very high risk of being inflicted with a severe form of HPV disease, as specified in the accompanying Examples.

The term "subject" as used herein relates to animals, preferably mammals, and, more preferably, humans. However, it is envisaged in accordance with the aforementioned method of the present invention that the subject shall be infected with at least two genotypes of HR-HPV. How to assess whether a subject is infected with HR-HPV is well known in the art. E.g., HR-HPV infection can be assessed by genotyping HR-HPV DNA in a sample of a subject by Southern and dot blot hybridisation, in situ hybridisation, by signal amplification assays, or by various PCR methods (Molijn, A., B. Kleter, W. Quint, and L. J. van Doom. 2005. Molecular diagnosis of human papillomavirus (HPV) infections. J Clin Virol 32 Suppl 1:S43-51). The skilled artisan knows how to select one of the methods supra suited to detect an infection of a subject by more than one HR-HPV genotype. Preferably, the method is a multiplex method, more preferably the method of WO/2009/027403. A "subject infected with at least two HR-HPV genotypes" is a subject carrying HR-HPV viruses or detectable parts thereof on or within his body. It is to be understood that step a) of the method of the current invention can be applied to a sample from any subject, whether infected with HPV or not. But only if in the quantification genome numbers of two HR-HPV genotypes above cutoff are detected, steps b) and c) can be applied. Preferably, the method of the invention is applied to a sample from a subject suspected to be infected with HPV, more preferably a subject suspected to be infected with HR-HPV.

The term "human papillomavirus" or HPV relates to human-infecting members of the family of non-enveloped, dsDNS viruses generally known as Papillomaviridae. Preferably, the term relates to genotypes infecting the human mucosa. The term high-risk (HR)-HPV relates to genotypes infecting the human mucosa and associated with cancer. Preferably, the term HR-HPV includes possibly high-risk genotypes, e.g. HPV 26, 53, 67, 70, 73 and 82 and high-risk HPV genotypes. More preferably, HR-HPV are high-risk genotypes and include HPV 16, 18, 26, 31, 33, 35, 39, 45, 51, 52, 53, 56, 58, 59, 66, 67 and 68. Most preferably, HR-HPV genotypes are HPV 16, 18, 26, 31, 33, 35, 39, 45, 51, 52, 53, 56, 58, 59, 66, 67, 68, 70, 73 and 82.

The term "severe form of HPV infection" relates to a form of HR-HPV infection that is histologically classified as intraepithelial neoplasia, preferably cervical intraepithelial neoplasia (CIN), including all stages (CIN1, CIN2 (moderate cervical epithelial dysplasia), CIN3 (severe cervical dysplasia), and cervix carcinoma (in situ or invasive). Accordingly, the term "severe form of HPV infection" preferably, refers to a form of HR-HPV infection that is cytologically classified as LSIL, HSIL or cancer. Thus, the severe form of HPV infection, preferably, encompasses malign cervical lesions, and, thus, high-grade HPV lesions (for a review see Smith, J. H. 2002. Bethesda 2001. Cytopathology 13:4-10).

The term "mild form of HPV infection" relates to a form of HR-HPV infection wherein the subject is infected with HR-HPV without showing apparent signs of disorder related to said infection. Preferably, in a mild form of HPV infection a sample is classified as normal in cytological and / or histological examination.

As used herein, the term "quantifying the genome numbers" relates to determining or measuring the number of HPV genomes present in a sample; quantification preferably is absolute, i.e. relating to a specific number of genome molecules, or, more preferably, relative, i.e. measured in arbitrary units. Methods allowing for absolute or relative quantification of HPV genomes are well known in the art. E.g., quantitative PCR methods are methods for relative quantification; if a calibration curve is incorporated in such an assay, the relative quantification can be used to obtain an absolute quantification. Other methods known are, e.g. nucleic acid sequence-based amplification (NASBA) or the Branched DNA Signal Amplification Assay method in combination with dot blot or Luminex detection of amplified polynucleotides. It is to be understood that quantifying genome numbers also relates to a semiquantitative quantification, wherein samples are assigned to one of at least two, at least three, at least four, or at least five categories of genome quantity.

Preferably, the genome numbers are quantified by normalizing the amplification products obtained from the genomes , i.e. the genome derived amplification products are are set into relation to at least one reference amplification product, thereby, preferably, setting the genome numbers into relation to the number of cells in the sample and / or the efficiency of polynucleotide amplification. Thus, preferably, the reference amplification product is a product obtained from a polynucleotide known to have a constant abundancy in each cell, i.e. a polynucleotide comprised in most, preferably all, cells of a sample in approximately the same amount. More preferably, the reference amplification product is amplified from a chromosomal or mitochondrial gene or from the mRNA of a housekeeping gene. Most preferably, the reference amplification product is amplified from the beta-globin DNA.

More preferably, the genome numbers are furthermore normalized by setting the normalized genome derived amplification products as defined supra for each HPV genotype into relation to the maximum amount of amplification products obtainable for said HPV genotype. Preferably, said maximum amount of amplification products is obtained in a corresponding separate reference amplification reaction using the same oligonucleotide primers and PCR conditions except that a suitable positive control polynucleotide is used in an amount high enough to ensure the synthesis of the maximum amount of amplification products. The skilled artisan knows how to select the positive control polynucleotide and a suitable amount thereof; an example can be found in the accompanying Examples.

The term "sample" refers to a sample of a body fluid, to a sample of separated cells or to a sample from a tissue or an organ or to a sample of wash/rinse fluid obtained from an outer or inner body surface. The sample comprises polynucleotides, preferably the sample comprises DNA. Samples can be obtained by well known techniques and include, preferably, scrapes or biopsies from the urogenital tract, perianal regions, anal canal, the oral cavity, the upper aerodigestive tract and the epidermis. Such samples can be obtained by use of brushes, (cotton) swabs, spatula, rinse/wash fluids, punch biopsy devices, puncture of cavities with needles or surgical instrumentation. Preferably, the sample contains mucosal cells, more preferably the sample comprises mucosal cells from the cervix. However, samples of blood, plasma, serum, urine, saliva, lacrimal fluid, stool are also encompassed by the method of the present invention. Tissue or organ samples may be obtained from any tissue or organ by, e.g., biopsy or other surgical procedures. Separated cells may be obtained from the body fluids or the tissues or organs by separating techniques such as filtration, centrifugation or cell sorting. Preferably, cell, tissue or organ samples are obtained from those cells, tissues or organs which are known or suspected targets of mucosal HPV genotypes, more preferably of HR-HPV genotypes, and, therefore, may comprise HPV-specific polynucleotides. It is to be understood that the sample may be further processed in order to carry out the method of the present invention. Particularly, polynucleotides such as DNA or RNA, preferably DNA, might be extracted and/or purified from the obtained sample by methods and means known in the art (e.g., see Examples). Thus, the term sample also may relate to polynucleotides, preferably DNA, purified and/or extracted from any sample as mentioned above.

The term "comparing" as used herein encompasses comparing the genome number determined in step a) of the methods of the present invention to a suitable reference amount, the source of which is specified elsewhere in this description. It is to be understood that comparing as used herein refers to a comparison of amounts. The comparison referred to in step b) of the methods of the present invention may be carried out manually or computer-assisted. For a computer-assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the genome numbers to a reference amount it is possible to differentiate, in a subject infected with at least two HR-HPV genotypes, between a mild form of HPV infection and a severe form of HPV infection. Therefore, the reference is to be chosen so that either a difference or a similarity in the compared amounts allows for differentiating between a severe and a mild form of HPV infection.

Accordingly, the term "reference", "reference amount" or "reference value" as used herein, preferably, refers to an amount or value which allows differentiation between a mild form and a severe form of HPV infection. Accordingly, the reference may be derived from carrying out step a) of the methods of the present invention in samples from subjects being known to suffer from a severe form of HPV infection such as LSIL, HSIL or cervical cancer. Also, the reference may be derived from carrying out steps a) of the methods of the present invention in samples from subjects being known to suffer from a mild form of HPV infection. Moreover, the reference amounts, preferably, define thresholds. Suitable reference amounts or thresholds may be determined by the method of the present invention from a reference sample to be analyzed together, i.e. simultaneously or subsequently, with the test sample.

It is to be understood that a reference amount, preferably, is specific for a specific HR-HPV genotype, i.e. different genotypes may necessitate the use of different reference amounts. Thus, preferably, e.g. for two HR-HPV genotypes, two reference amounts are determined; for three HR-HPV- genotypes, three reference amounts are determined; for five HR-HPV- genotypes, five reference amounts are determined, etc. More preferably, genome numbers are double normalized such that a single reference amount can be applied to all HR-HPV genotypes. Said double normalization most preferably is achieved by the methods described herein.

It is also to be understood that the value of the reference or threshold may vary depending on the nature of the target gene and depending on how the amount of a gene product is determined in the sample. For example, if the determination of the amount of the gene product includes amplification of the gene product by PCR (polymerase chain reaction), the determined amount of a gene product may depend, e.g., on the oligonucleotides used for the PCR reaction since the amplification efficiency of various oligonucleotide pairs for the amplification of a specific gene product varies. However, the person skilled in the art considers this when calculating the reference amount. Particularly, the person skilled knows that, preferably, the same means and methods have to be used for determining the amounts of a specific gene product in a reference sample and in a test sample.

A preferred reference amount serving as a threshold may be derived from the upper limit of normal (ULN), i.e. the upper limit of the physiological amount to be found in a population of healthy subjects. The ULN for a given population of subjects can be determined by various well known techniques. More preferably, the reference amount is derived from a population having a mild form of HPV disease. Moreover, a suitable cutoff can be calculated by means known in the art. Specifically, the accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa). Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from a severe form of HPV infection or those which suffer from a mild form of HPV infection among a cohort of subjects suffering from HPV infection can be generated, preferably, by establishing a ROC for said cohort as described above and deriving a threshold amount therefrom.

Preferably, a genome number in the test sample higher than the reference indicates a severe form of HPV infection. More preferably, a genome number in the test sample significantly higher than the reference indicates a severe form of HPV infection. Most preferably, a genome number in the test sample that is statistically significantly higher than the reference indicates a severe form of HPV infection. Preferred references indicating a severe form of HPV infection can be derived from a subject or a group of subjects known to suffer from said severe form of HPV infection.

Preferably, a genome number in the test sample lower than the reference indicates a mild form of HPV infection. More preferably, a genome number in the test sample significantly lower than the reference indicates a mild form of HPV infection. Most preferably, said genome number is statistically significantly lower than the reference. Preferred references indicating a mild form of HPV infection can be derived from a subject or a group of subjects known to suffer from said mild form of HPV infection.

Whether an amount is statistically significantly higher (or lower) can be determined without further ado by the person skilled in the art using various well known statistic evaluation tools.

Preferably, the reference is selected such that less than 30%, less than 25%, less than 20%, less than 15%, less than 10%, less than 7%, less than 5%, less than 3%, less than 1%, or less than 0.5% of subjects not suffering from a severe form of HPV disease are diagnosed to suffer from a severe form of HPV disease and such that less than 20%, less than 15%, less than 10%, less than 7%, less than 5%, less than 3%, less than 1%, or less than 0.5% of subjects suffering from a severe form of HPV infection are diagnosed not to suffer from a severe form of HPV infection. More preferably, the reference amount is selected such that less than 20% of subjects not suffering from a severe form of HPV disease are diagnosed to suffer from a severe form of HPV disease and such that less than 10% of subjects suffering from a severe form of HPV infection are diagnosed not to suffer from a severe form of HPV infection. Most preferably, the reference amount is selected such that less than 10% of subjects not suffering from a severe form of HPV disease are diagnosed to suffer from a severe form of HPV disease and such that less than 5% of subjects suffering from a severe form of HPV infection are diagnosed not to suffer from a severe form of HPV infection.

As used herein, the term "HR-HPV genotype with the highest genome number" relates to the HR-HPV genotype for which the quantification of genome numbers of step a) of the methods of the current invention results in the highest amount. Determination of the highest amount ist straightforward if genome numbers are determined as absolute genome numbers, which are comparable without further ado. However, the skilled artisan also knows which further steps need to be applied to the methods of the current invention in order to allow comparison, i.e. determination of the highest amount, of genome numbers of different HR-HPV genotypes in case relative genome numbers are determined. Preferably, these further steps include, e.g. performing the quantification step on separate control samples with known genome numbers and comparing the relative genome numbers of the samples thereto.

More preferably, the HR-HPV genotype with the highest genome number is determined on the basis of double normalized genome numbers as described herein above and in the accompanying Examples.

It is to be understood that methods which by their design do not allow differentiation of the contribution of individual HR-HPV genotypes to the output genome number, like, e.g., the hybrid capture assay, are not suited for the method of the current invention.

According to the data presented in the present invention, it is possible to make a clinically useful assessment of HPV-associated disease based on genome numbers of HR-HPV genotypes indicative of the stage of HPV-associated disease. This method is technically simple and, in a preferred embodiment, is amenable to automation in a high-throughput format.

Thus, the method of the present invention is particularly advantageous since it allows for rapid, reliable, inexpensive differentiation between mild and severe forms of HPV infection. Particularly, the method allows for identifying those subjects which are at elevated risk and, thus, which are in need for a cancer therapy and/or being susceptible to a cancer examination, particularly colposcopical and histological examination and potentially therapy. Moreover, the method allows for identifying those subjects suffering only from mild forms of HPV infection, and which are not in need for additional intensive examination and therapy. Without applying the method of the present invention, the said subjects might have been examined and treated too much (resulting in an increased risk of adverse side effects and increased health care cost).

The definitions made above apply mutatis mutandis to the following.

In another preferred embodiment, the current invention relates to a method for diagnosing a severe HPV disease in a subject comprising the method of the current invention described above.

The term "diagnosing" as used herein refers to assessing the probability according to which a subject is suffering or will suffer from a disease or condition referred to in this specification. As will be understood by those skilled in the art, such an assessment is usually not intended to be correct for 100% of the subjects to be diagnosed. The term, however, requires that a statistically significant portion of subjects can be correctly diagnosed to suffer from the disease or condition. Whether a portion is statistically significant can be determined by the methods described herein above.

The current invention also relates to an oligonucleotide specifically hybridizing to a polynucleic acid from a specific HR-HPV-genotype, for use in a diagnostic method according to this specification.

An "oligonucleotide" in the context of the present invention, preferably, is a single-stranded nucleic acid molecule that specifically hybridizes with the sense strand or the antisense strand of at least one genome of the HR-HPV genotypes of the current invention. Thus, an oligonucleotide comprises a stretch of nucleotides that specifically hybridize with the sense strand or the antisense strand of said at least one HR-HPV genome. Said stretch of nucleotides is, preferably, 85%, 90%, 95%, 99% or, more preferably, 100% identical to the sense strand or the antisense strand of one of said at least one HR-HPV genomes. The percent identity values are, preferably, calculated over the entire nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

Preferably, a specific oligonucleotide is a specific indicator for the HPV genotype associated with the nucleotide sequence. Preferably, an oligonucleotide as meant herein is between 10 and 50 nucleotides in length, more preferably said oligonucleotide is between 15 and 30 nucleotides in length, and most preferably between 18 and 23 nucleotides in length. Preferably, the oligonucleotides are bound to a carrier providing a solid surface. More preferably, said carrier is a small particle or bead. The overall size of a small particle or bead, preferably, may be in the micrometer or nanometer range. Preferably, said beads and particles may be stained with a specific dye, more preferably with a specific fluorescent dye. Preferably, by staining various carriers with various dyes, the carriers can be distinguished from each other. By using a carrier with a specific dye for a specific probe oligonucleotide (thus, a nucleic acid that targets the amplified polynucleotides of a specific low-risk HPV type), said carrier is distinguishable from other carriers comprising different dyes. In one preferred embodiment, commercially available Luminex microspheres (Luminex Corp., Austin, Texas, USA) are used. Thus, for determining HR- HPV types, the HR- HPV type-specific oligonucleotides are coupled to fluorescence-labelled polystyrene beads (Luminex suspension array technology) which are hybridized with amplification products as specified herein above under suitable, preferably, stringent conditions. It is, however, also contemplated by the current invention that the oligonucleotides are linked to a suitable carrier in a spatially separated way, e.g. in the form of microarrays, Reverse-Line blots (RLB), dot blots or similar technologies.

Preferably, probe oligonucleotides are used having a sequence as disclosed in WO 2009/027403

The oligonucleotides of the present invention may be labelled or contain other modifications which allow a detection and/or analysis of a hybridization product and/or the binding to a carrier. Labelling can be done by various techniques well known in the art and depending of the label to be used. Particularly, the oligonucleotides may be biotinylated in order to enable binding to a streptavidin surface or fluorescent conjugate. Exemplary labels to be used in the context of the present invention are, but are not limited to, fluorescent labels comprising, inter alia, fluorochromes such as R-phycoerythrin, Cy3, Cy5, fluorescein, rhodamin, Alexa, or Texas Red. However, the label may also be an enzyme or an antibody. It is envisaged that an enzyme to be used as a label will generate a detectable signal by reacting with a substrate. Suitable enzymes, substrates and techniques are well known in the art. An antibody to be used as label may specifically recognize a target molecule which can be detected directly (e.g., a target molecule which is itself fluorescent) or indirectly (e.g., a target molecule which generates a detectable signal, such as an enzyme). The oligonucleotides of the present invention may also contain 5' restriction sites, locked nucleic acid molecules (LNA) or be part of a peptide nucleotide acid molecule (PNA). Such PNA can be, in principle, detected via the peptide part by, e. g., antibodies.

It is, however, also contemplated by the current invention that the oligonucleotides are primer oligonucleotides specifically priming enzymatic polynucleotide polymerization, e.g. PCR amplification, with at least one HR-HPV genotype genome as a template. Preferably, the primer oigonucleotides allow for specific priming of enzymatic polynucleotide polymerization with more than one HR-HPV genotype as template, specific priming relating to the virtual absence of priming on non-HR-HPV templates. Preferably, primer oligonucleotides are used having a sequence as disclosed in WO 2009/027403.

In another preferred embodiment, the current invention relates to a device for diagnosing whether a subject infected with at least two HR-HPV genotypes is at increased risk of suffering from severe HPV-disease, comprising a) an analysis unit quantifying the genome numbers of each of said at least two HR-HPV-genotypes in a sample from said patient and calculating genome numbers, and b) an evaluation unit comprising a computer implemented algorithm for comparing the detected genome numbers from the analyzing unit to a stored reference amount.

The term "device" as used herein relates to a system comprising the aforementioned units operatively linked to each other as to allow the diagnosis or monitoring according to the methods of the invention. The term "analysis unit" as used herein refers to an agent which is capable of specifically quantifying HR-HPV genotypes present in a sample. Preferably, HPV genomes are amplified by one of the methods described herein before quantification occurs. Preferred detection methods are detection of luminescence, fluorescence, or absorbance. The determined amount of genomes can be transmitted to the evaluation unit. Said evaluation unit comprises a data processing element, such as a computer, with an implemented algorithm for carrying out a comparison between the determined amount and a suitable reference. It is to be understood that the data obtained will need interpretation by the clinician. However, also envisaged are expert system devices wherein the output comprises processed diagnostic raw data the interpretation of which does not require a specialized clinician. Preferably, the evaluation unit further comprises a computer implemented algorithm for selection of one of the genome numbers for comparing to said stored reference amount.

All references cited in this specification are herewith incorporated by reference with respect to their entire disclosure content and the disclosure content specifically mentioned in this specification.

### FIGURES

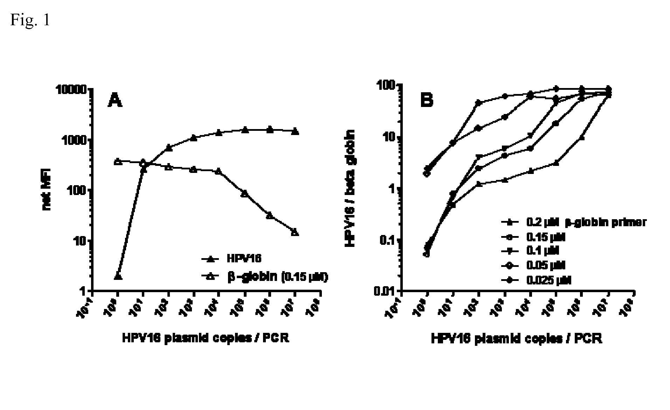

**Figure 1** shows HPV 16 quantification by net MFI [A] or HPV 16 to beta-globin ratios [B]in HPV16 plasmid dilution series in 100 ng human placenta DNA per PCR.

The following Examples shall merely illustrate the invention. They shall not be construed, whatsoever, to limit the scope of the invention.

### EXAMPLES

### Example 1: Quantification of HPV subtypes by BSGPS+/6+-PCR/MPG

The detection of 51 HPV types and three subtypes, including Hr-HPV types 16, 18, 31, 33, 35, 39, 45, 51, 52, 56, 58, 59, 66, 68a/b and pHr-HPV types 26, 53, 67, 70, 73, 82, and beta-globin by the BSGP5+/6+-PCR/MPG assay was performed as described with some modifications; see WO2009/027403. HPV amplification was carried out using the Multiplex PCR Kit (Qiagen, Hilden, Germany). 0.2 to 0.5µM each of the BSGP5+ and 5'-biotinylated BSGP6+ primers, and 0.15µM each of the beta-globin primers MS3 and 5'biotinylated MS10 were added to the PCR mixture. The cutoff value (5 net Median Fluorescence Intensities (MFI)) to define HPV DNA positivity was applied as described previously; see WO2009/027403. For the colony PCR, Escherichia coli DH5α, transformed by high-copy-number plasmids containing the viral genome, replaced the template DNA. Depending on the amount of transferred bacteria, colony PCRs are estimated to contain >10⁶ HPV copies per PCR. Quantification of HPV signals was accomplished by computing for each positive reaction the relative HPV MFI signal (%) by dividing the measured HPV MFI value with the maximum value detected of this HPV type using colony PCR products. Finally, the relative MFI (%) was divided by the measured beta-globin MFI value to form a non-descriptive viral load value (BS viral load) (%HPVMFI / beta-globin MFI).

BSGP5+/6+-PCR/MPG allowed the semi-quantitative analysis of HPV containing plasmid dilution series between 10 and 10,000 copies per PCR (Schmitt et al., 2008). Due to amplification competition of beta-globin with HPV, it was further observed that specimens with high viral loads showed reduced amplification of beta-globin (unpublished data). In order to strengthen this effect, beta-globin primer concentrations were titrated during the amplification of 10-fold dilution series of HPV16 containing plasmid DNA (Fig. 1). While HPV16 net MFI values reached the plateau phase with 1,000 to 10,000 copies per PCR, beta-globin signals declined with increased HPV copy number input (Fig. 1). The HPV16/beta-globin ratios, however, were able to quantify over 7 logs when beta-globin primer concentrations between 0.1 and 0.2 µM were used.

### Example 2: Risk of cytological abnormality associated with multiple high viral load

DNA aliquots of 1,000 consecutive samples, collected during routine gynaecological health checks from women in Flanders (Belgium), and 100 smears each from patients with ASC-US, LSIL and HSIL were analysed by the BSGP5+/6+-PCR-MPG assay. Multiple infections were most prevalent in LSIL (75.9% of HPV-positive smears), followed by HSIL (65.5%), ASC-US (64.6) and NIL/M (36.8%) (Table 1). In a single ASC-US sample, 17 concurrent HPV genotypes could be detected. Restricting the analysis to high viral load infections, there was a reduction in the number of multiple infections, however, the order remained the same with LSIL (62.6%), followed by HSIL (51.9%), ASC-US (40.7) and NIL/M (19.3%). Multiple high viral load infections with up to 7 (HSIL), 6 (ASC-US) and 12 concurrent HPV types (LSIL) could be detected. In contrast, in NIL/M only triple high viral load infections were observed. Age adjusted OR showed a significant positive association of multiple infections with the presence of LSIL and HSIL compared to NIL/M, which was stronger for multiple high viral load infections then for multiple DNA positivity alone (Table 1). Patients with multiple high viral loads showed a 5- to 6-fold increased risk of having cervical lesions than patients with single high viral loads.

**Table 1: Age adjusted Odd Ratios for disease categories associated with multiple infection compared to single infections or multiple high viral loads compared to single high viral loads.**

| **Assay** | **Disease category** | **Multiple infection ¹** | **OR (95% CI)** | |
|---|---|---|---|---|
| | | | **Positivity** | **High load** |
| BSGP5+/6+-PCR/MPG | HSIL vs NIL/M | single infection | 1.0 (reference) | 1.0 (reference) |
| | | 14 | **1.78 (1.08 - 2.95)** | **5.81 (1.90 - 17.8)** |
| | | 20 | **2.63 (1.62 - 4.25)** | **5.28 (2.16 - 12.9)** |
| | | 54 | **3.00 (1.89 - 4.76)** | **4.37 (2.35 - 8.13)** |
| | LSIL vs NIL/M | single infection | 1.0 (reference) | 1.0 (reference) |
| | | 14 | **2.35 (1.41 - 3.93)** | **6.58 (2.14 - 20.2)** |
| | | 20 | **2.60 (1.59 - 4.26)** | **5.82 (2.38 - 14.2)** |
| | | 54 | **4.74 (2.87 - 7.83)** | **6.39 (3.43 - 11.9)** |
| | HSIL vs LSIL | single infection | 1.0 (reference) | 1.0 (reference) |
| | | 14 | 0.77 (0.44 - 1.35) | 0.88 (0.48 - 1.60) |
| | | 20 | 1.03 (0.59 - 1.79) | 0.91 (0.52 - 1.62) |
| | | 54 | 0.66 (0.36 - 1.21) | 0.70 (0.40 - 1.24) |
| | CIN3 vs NIL/M | single infection | 1.0 (reference) | 1.0 (reference) |
| | | 14 | 1.57 (0.78 - 3.16) | **4.22 (1.23 - 14.5)** |
| | | 20 | **2.16 (1.09 - 4.26)** | **4.15 (1.50 - 11.5)** |
| | | 54 | **2.86 (1.45 - 5.65)** | **4.12 (1.88 - 9.02)** |
| | CIN2+ vs NIL/M | single infection | 1.0 (reference) | 1.0 (reference) |
| | | 14 | 1.71 (0.94 - 3.11) | **4.77 (1.49 - 15.2)** |
| | | 20 | **2.58 (1.44 - 4.62)** | **5.16 (2.01 - 13.2)** |
| | | 54 | **3.20 (1.79 - 5.71)** | **4.82 (2.38 - 9.76)** |
| | CIN2 vs NIL/M | single infection | 1.0 (reference) | 1.0 (reference) |
| | | 14 | 2.17 (0.88 - 5.40) | **7.03 (1.78 - 27.7)** |
| | | 20 | **4.07 (1.57 - 10.6)** | **9.10 (2.78 - 29.8)** |
| | | 54 | **4.17 (1.57 - 11.1)** | **6.58 (2.39 - 18.1)** |
| | CIN1 vs NIL/M | single infection | 1.0 (reference) | 1.0 (reference) |
| | | 14 | 2.16 (0.92 - 5.09) | **5.64 (1.37 - 23.2)** |
| | | 20 | **2.56 (1.09 - 6.00)** | **9.38 (2.87 - 30.6)** |
| | | 54 | **4.14 (1.67 - 10.3)** | **7.48 (2.83 - 19.8)** |
| | CIN2+ vs CIN1 | single infection | 1.0 (reference) | 1.0 (reference) |
| | | 14 | 0.79 (0.30 - 2.07) | 1.05 (0.38 - 2.89) |
| | | 20 | 1.01 (0.37 - 2.73) | 0.67 (0.26 - 1.75) |
| | | 54 | 0.71 (0.24 - 2.12) | 0.61 (0.22 - 1.70) |
| | CIN3+ vs CIN1 | single infection | 1.0 (reference) | 1.0 (reference) |
| | | 14 | 0.71 (0.25 - 1.98) | 0.85 (0.28 - 2.56) |
| | | 20 | 0.82 (0.28 - 2.37) | 0.50 (0.17 - 1.42) |
| | | 54 | 0.64 (0.20 - 2.04) | 0.52 (0.17 - 1.57) |

| | | | | |
|---|---|---|---|---|
| ¹ number of HPV types analysed: 14: multiple infections with the 14 HPV types included in GP5+/6+-PCR/EIA; 20: additional types 26, 53, 67, 70, 73 and 82 included according to IARC Monograph V. 100B. | | | | |

## Claims

1. A method for differentiating in a subject infected with at least two high-risk (HR-) human papillomavirus (HPV) genotypes between a severe form of HPV infection and a mild form of HPV infection, comprising
a) quantifying the genome numbers of each of said at least two HR-HPV-genotypes in a sample from said patient,
b) comparing the genome number of the HR-HPV genotype with the highest genome number to at least one reference, and
c) differentiating between (i) a severe form of HPV infection and (ii) a mild form of HPV infection.

2. The method of claim 1, wherein normalized genome numbers are calculated from genome numbers by setting the genome numbers of step a) into relation to at least one reference amplification product and wherein normalized genome numbers are used in steps b) and c).

3. The method of claim 2, wherein double normalized genome numbers are calculated from normalized genome numbers by setting the normalized genome numbers for each HR-HPV genotype of claim 2 into relation to the maximum signal obtainable for said HR-HPV genotype and wherein double normalized genome numbers are used in steps b) and c).

4. The method of any one of claims 1 to 3, wherein a quantitative PCR (qPCR), a Nucleic Acid Sequence Based Amplification (NASBA), or a Branched DNA Signal Amplification Assay method are used to quantify the numbers of HR-HPV genomes.

5. The method of any one of claims 1 to 4, wherein said at least two HR-HPV-genotypes are selected from the list consisting of HPV 16, 18, 26, 31, 33, 35, 39, 45, 51, 52, 53, 56, 58, 59, 66, 67, 68, 70, 73 and 82.

6. The method of any one of claims 1 to 5, wherein the reference amount is selected such that less than 30% of subjects not suffering from severe HPV disease are estimated to be at high risk and such that less than 20% of subjects suffering from severe HPV disease are estimated not to be at high risk to suffer from severe HPV disease.

7. The method of any one of claims 1 to 6, wherein specific reference amounts are calculated for at least two of said HR-HPV-genotypes.

8. The method of any one of claims 1 to 7, wherein genome numbers of all HR-HPV genotypes detected are compared to at least one reference amount in step b) and wherein a genome number higher than the reference amount for at least two HR-HPV-genotypes is indicative of a very high risk of suffering from severe HPV-related disease.

9. A method for diagnosing a severe form of HPV infection in a subject comprising the method of any one of claims 1 to 8.

10. The method of any one of claims 1 to 9 wherein said severe form of HPV infection is cervical cancer, a grade 2 cervical intraepithelial lesion (CIN2), or a grade 1 cervical intraepithelial lesion (CIN 1).

11. An oligonucleotide specifically hybridizing to a polynucleic acid from a specific HR-HPV-genotype, for use in a diagnostic method according to claim 9.

12. A device for diagnosing whether a subject infected with at least two HR-HPV genotypes is at increased risk of suffering from severe HPV-disease according to the method of any one of claims 1 to 10, comprising
a) an analysis unit quantifying the genome numbers of each of said at least two HR-HPV-genotypes in a sample from said patient and calculating genome numbers, and
b) an evaluation unit comprising a computer implemented algorithm for comparing the detected genome numbers from the analyzing unit to a stored reference amount.

13. The device of claim 12 wherein the evaluation unit of b) further comprises a computer implemented algorithm for selection of one of the genome numbers for comparing to said stored reference amount.
